# EUROPÄISCHE PATENTSCHRIFT

(11) **EP 2 544 648 B1**
(45) Veröffentlichungstag und Bekanntmachung des Hinweises auf die Patenterteilung: **18.05.2016**
(21) Anmeldenummer: 11704397.6
(22) Anmeldetag: 10.02.2011
(51) Int. Cl.: A61J 1/20, A61M 5/19, A61M 5/178, A61B 17/00

(54) **MODULARE BEFÜLLVORRICHTUNG FÜR EINEN APPLIKATOR**
MODULAR FILLING DEVICE FOR AN APPLICATOR
DISPOSITIF DE REMPLISSAGE MODULAIRE POUR UN APPLICATEUR

(30) Priorität: 10.03.2010 CH 3312010
(43) Veröffentlichungstag der Anmeldung: 16.01.2013
(73) Patentinhaber: Medmix Systems AG, 6343 Rotkreuz (CH)
(72) Erfinder: MATHYS, Beat, CH-5630 Muri (CH); KAYSER, Ralph, Egon, CH-6004 Luzern (CH)
(74) Vertreter: Detken, Andreas
(86) Internationale Anmeldenummer: PCT/CH2011/000023
(87) Internationale Veröffentlichungsnummer: WO 2011/109915

(56) Entgegenhaltungen:
- WO-A1-96/29113
- US-A1- 2005 209 555
- US-B1- 6 488 650

## Beschreibung

### TECHNISCHES GEBIET

Die vorliegende Erfindung betrifft eine Befüllvorrichtung, um einen Applikator mit mindestens einem Fluid zu befüllen. Insbesondere betrifft die Erfindung eine modulare Befüllvorrichtung, die es ermöglicht, einen Applikator je nach Bedarf aus verschiedenartigen Behältern zu befüllen. Gemäss einem weiteren Aspekt betrifft die vorliegende Erfindung ein Set aus mindestens einem Applikatorhalter und mehreren Behälterhaltem, wobei die Behälterhalter dazu ausgebildet sind, verschiedenartige Behälter zu halten.

### STAND DER TECHNIK

In verschiedenen Anwendungen stellt sich die Aufgabe, eine Mischung aus zwei oder mehr fliessfähigen Komponenten in einem vorgegebenen Mischverhältnis herzustellen und auszutragen. Ein Beispiel ist die Herstellung eines Klebemittels für technische oder medizinische Anwendungen, z.B. eines medizinischen Klebers auf Fibrinbasis. Ein anderes Beispiel ist die Herstellung eines Knochenzements aus mehreren Komponenten unter Verwendung eines Monomers. Auch gibt es z.B. Medikamente, die durch Mischen von zwei oder mehr Komponenten hergestellt werden, die im gemischten Zustand aber nicht lagerfähig sind. In diesem Fall ist es wünschenswert, die Komponenten zunächst getrennt zu lagern und erst unmittelbar vor ihrer Verabreichung zu mischen. Ähnliche Aufgaben stellen sich auch bei anderen pharmazeutischen oder chemischen Systemen aus zwei oder mehr Komponenten, die im gemischten Zustand nicht stabil sind.

Aus dem Stand der Technik ist es hierzu bekannt, die zu mischenden Komponenten getrennt in zwei Reservoirs eines Applikators, z.B. in Form einer Doppelspritze, aufzunehmen und aus diesem durch eine geeignete Mischvorrichtung hindurch auszutragen. Allerdings ist es häufig problematisch, fliessfähige Substanzen über einen längeren Zeitraum in Applikatoren aus Kunststoff zu lagern, da einerseits die Substanzen mit dem Kunststoff chemisch reagieren können, und da andererseits die Gefahr besteht, dass Gase, insbesondere Luftsauerstoff, durch die Wände des Applikators oder durch Dichtstellen hindurch diffundieren und den Inhalt chemisch verändern. Dies gilt in besonderem Masse für Anwendungen im medizinischen Bereich, wo die chemische Reinheit von besonderer Bedeutung ist.

Es ist daher bekannt, die zu mischenden Komponenten getrennt in sogenannten Vials zu lagern, insbesondere in Glasvials mit Septumverschluss, d.h. in sterilisierbaren Glasfläschchen, die an einem Ende durch eine durchstechbare, selbstverschiessende Membran (ein sogenanntes Septum) verschlossen sind, um die zu mischenden Komponenten erst kurz vor der Applikation aus den Vials in zwei getrennte Reservoirs aufzunehmen. Hierzu sind im Stand der Technik adapterartige Vorrichtungen vorgeschlagen worden, die ein simultanes Befüllen von zwei Reservoirs aus zwei Vials ermöglichen, z.B. die in US 6,610,033, US 6,488,650 offenbarten Befüllvorrichtungen.

Auch die WO 2009/144085 offenbart eine derartige Befüllvorrichtung. Hier sind zwei Vialhalter für jeweils ein Vial mit einem Applikatorhalter verbunden. Ein Applikator ist entlang einer Befestigungsrichtung in den Applikatorhalter einsteckbar und über Luerverbindungen mit dem Applikatorhalter verbindbar. Die Vialhalter sind von der entgegengesetzten Seite her in die Behälteraufnahmen einschiebbar oder einschraubbar. Durch Fluidverbindungen im Applikatorhalter hindurch kann der Applikator aus den Vials befüllt werden. Diese Befüllvorrichtung erlaubt nur das Befüllen aus Vials, nicht jedoch aus andersartigen Behältern.

Aus der EP 1 454 650 A1 ist eine Transfervorrichtung bekannt, welche ein Gehäuse mit zwei in diesem gelagerten Einstechelemente zum Einstechen von in das Gehäuse eingesetzten Glasfläschchen und einen zwischen diesen angeordneten Schieber aufweist. Der Schieber ist zu den Einstechelementen verschiebbar, wobei in einer ersten Verschiebestellung eine Strömungsverbindung zwischen den beiden Einstechelementen und in einer zweiten Verschiebestellung eine Strömungsverbindung zwischen einem der Einstechelemente und einer seitlichen Öffnung des Gehäuses gebildet ist.

Statt in Vials können derartige Komponenten aber auch in anderen Behältern aufgenommen sein, z.B. in Glasampullen, d.h. hermetisch verschlossenen Glasgefässen, die zur Entnahme der darin enthaltenen Komponente aufgebrochen werden müssen, oder in Tuben mit verformbarem Wandbereich. Es ist auch denkbar, eine der Komponenten in einer Spritze zu lagern, sofern die betreffende Komponente nicht allzu empfindlich gegenüber Umwelteinflüssen ist, und sie erst kurz vor der Anwendung in den eigentlichen Applikator aufzunehmen.

### DARSTELLUNG DER ERFINDUNG

Gemäss einem ersten Aspekt stellt die vorliegende Erfindung eine Befüllvorrichtung zur Verfügung, die es ermöglicht, mindestens ein Reservoir eines Applikators aus einem Behälter mit einem Fluid zu befüllen, wobei die Vorrichtung ohne wesentliche Konstruktionsänderungen an verschiedenartige Arten von Behältern anpassbar ist.

Eine derartige Befüllvorrichtung ist in Anspruch 1 angegeben. Weitere Ausführungsformen sind in den abhängigen Ansprüchen angegeben.

Es wird also eine Befüllvorrichtung zum Befüllen mindestens eines ersten Reservoirs eines Applikators mit einem Fluid aus mindestens einem ersten Behälter angegeben, wobei die Befüllvorrichtung aufweist:
einen ersten Behälterhalter mit einem ersten Haltebereich, der dazu ausgebildet ist, einen ersten Behälter am ersten Behälterhalter zu halten, wobei der erste Behälterhalter eine erste Auslassöffnung und einen Fluidkanal zwischen dem ersten Haltebereich und der ersten Auslassöffnung aufweist, um ein erstes Fluid durch die erste Auslassöffnung hindurch aus dem ersten Behälter zu entnehmen; und
einen Applikatorhalter mit einem Befestigungsbereich, der dazu ausgebildet ist, einen Applikator entlang einer Befestigungsrichtung lösbar am Applikatorhalter zu befestigen, und wobei der Applikatorhalter eine erste Einlassöffnung und eine erste Fluidverbindung zwischen der ersten Einlassöffnung und dem Befestigungsbereich aufweist, um das erste Fluid durch die erste Einlassöffnung in ein erstes Reservoir des Applikators aufzunehmen; und
wobei der erste Behälterhalter entlang einer ersten Verbindungsrichtung derart mit dem Applikatorhalter verbindbar ist, dass die erste Auslassöffnung und die erste Einlassöffnung miteinander kommunizieren.

Um zu erleichtern, dass die Behälterhalter für ganz unterschiedliche Arten von Behältern ausgestaltet werden können, z.B. für Vials, Tuben, Spritzen, Ampullen usw., verläuft die erste Verbindungsrichtung quer (gewinkelt mit einem Winkel von mehr als 45°, bevorzugt mehr als 60°, besonders bevorzugt im Wesentlichen senkrecht) zur Befestigungsrichtung für den Applikator. Vom Applikator aus betrachtet wird also der Behälterhalter von der Seite her mit dem Applikatorhalter verbunden.

Bevorzugt ist der Applikator durch eine reine Verschiebungsbewegung entlang der Befestigungsrichtung mit dem Applikatorhalter verbindbar, z.B. über eine Steckverbindung. Insbesondere ist der Applikator bevorzugt auf den Applikatorhalter aufschiebbar oder in ihn einschiebbar. Er kann jedoch zur Befestigung auch eine komplexere Bewegungsform, z.B. eine kombinierte Verschiebung und Drehung, wie z.B. bei einer Bajonettverbindung, ausführen. Der Translationsanteil der Bewegung definiert dann die Befestigungsrichtung. Die Verbindung des Applikators mit dem Applikatorhalter ist lösbar, um nach dem Befüllen den Applikator vom Applikatorhalter trennen zu können und das im Applikator aufgenommene Fluid austragen zu können, z.B. durch ein am Applikator befestigbares Zubehörteil wie eine Spraydüse, einen Mischer usw.

Auch der erste Behälterhalter ist bevorzugt durch eine reine Verschiebungsbewegung entlang der ersten Verbindungsrichtung mit dem Applikatorhalter verbindbar. Auch hier ist es bevorzugt, dass der erste Behälterhalter auf den Applikatorhalter aufschiebbar oder in diesen einschiebbar ist. Auch hier kann jedoch zur Befestigung eine komplexere Bewegungsform vorgesehen sein, deren Translationsanteil dann die erste Verbindungsrichtung definiert. Die Verbindung zwischen Behälterhalter und Applikatorhalter kann lösbar oder unlösbar (ohne Zerstörung) sein.

Der Behälterhalter und der Applikatorhalter bilden im verbundenen Zustand vorzugsweise eine im Wesentlichen starre Einheit. Es ist also keine flexible Schlauchverbindung oder dergleichen zwischen dem Behälterhalter und dem Applikatorhalter vorhanden, die die Handhabung erschweren würde. Selbstverständlich schliesst der Begriff "starre Einheit" aber nicht aus, dass der Behälterhalter oder der Applikatorhalter selbst bewegliche, auch flexible Teile aufweisen, z.B. zum Öffnen des Behälters. Der Begriff "starre Einheit" soll lediglich angeben, dass der Behälterhalter und der Applikatorhalter relativ zueinander eine definierte Orientierung einnehmen.

Der erste Behälterhalter wiederum ist bevorzugt so ausgestaltet, dass der erste Behälter entlang einer Behälterführungsrichtung am ersten Behälterhalter anbringbar ist, die quer zur ersten Verbindungsrichtung verläuft und die im Wesentlichen antiparallel zur Befestigungsrichtung für den Applikator verläuft, wenn der Applikatorhalter und der erste Behälterhalter miteinander verbunden sind. Die Handhabung der Befüllvorrichtung nach dem Verbinden des Applikatorhalters mit dem Behälterhalter erfolgt also vorzugsweise in der dem Benutzer vertrauten Form, indem der Behälter in der entgegengesetzten Richtung zum Applikator an der Befüllvorrichtung angebracht, z.B. eingeschoben, aufgeschoben, eingeschraubt usw. wird. Selbstverständlich kann der Behälter auch schon am Behälterhalter vormontiert sein, so dass zum Befüllen des Applikators nur noch die gesamte Einheit aus Behälterhalter und Behälter auf den Applikatorhalter aufgeschoben zu werden braucht.

Bei dem Behälter, der am ersten Behälterhalter gehalten ist, kann es sich z.B. eine Spritze mit einem Spritzenkörper und darin verschiebbarem Spritzenkolben, um eine Tube mit starrem distalem Verbindungsbereich, z.B. in Form eines Stutzens mit Aussengewinde, und flexiblem Seitenwandbereich, um ein Vial mit durchstechbarem Verschluss, insbesondere ein Glasvial mit Septumverschluss, oder um eine Ampulle, z.B. eine übliche Glasampulle mit Ampullenkörper, verjüngtem Hals und abbrechbarer Ampullenspitze handeln. Auch andere Arten von Behältern sind denkbar, z.B. Glasfläschchen mit Schraubanschluss usw. Je nach Behälter wird der Behälterhalter entsprechend andersartig ausgestaltet sein. Der vorgesehene Einsatzzweck definiert somit auch implizit die strukturelle Ausgestaltung des Behälterhalters in weiten Grenzen.

Konkret kann der Applikatorhalter insbesondere wie folgt ausgestaltet sein. Der Applikatorhalter umfasst einen Basiskörper mit einer Oberseite und einer Unterseite. Der Befestigungsbereich ist an der Unterseite des Basiskörpers angeordnet und kann insbesondere einstückig mit dem Basiskörper ausgebildet sein. Die erste Fluidverbindung umfasst einen sich im Basiskörper im Wesentlichen entlang der ersten Verbindungsrichtung von der ersten Einlassöffnung her erstreckenden ersten Einlassabschnitt und einen damit verbundenen, zum Befestigungsbereich führenden ersten Auslassabschnitt, wobei der erste Auslassabschnitt gewinkelt zum ersten Einlassabschnitt verläuft und sich in Richtung der Unterseite erstreckt. Vorzugsweise erstreckt sich der erste Auslassabschnitt im Wesentlichen senkrecht zum ersten Einlassabschnitt und verläuft insbesondere bevorzugt im Wesentlichen entlang der Befestigungsrichtung.

Der Basiskörper ist vorzugsweise ein im Wesentlichen flaches Gebilde, von dessen Unterseite her der Befestigungsbereich nach unten ragt. Dabei weist der Basiskörper vorzugsweise entlang der ersten Verbindungsrichtung eine Länge auf, die mindestens dem Dreifachen seiner Dicke entlang der Befestigungsrichtung entspricht. Die Breite quer zu diesen beiden Richtungen ist vorzugsweise mindestens das Doppelte der Höhe. Eine solche Gestaltung des Basiskörpers ermöglicht ein kompaktes Design. Selbstverständlich sind aber auch andere Formen des Basiskörpers möglich.

Der erste Behälterhalter ist bevorzugt entlang der ersten Verbindungsrichtung auf den Basiskörper aufschiebbar, um die erste Einlassöffnung mit der ersten Auslassöffnung zu verbinden, so dass der Behälterhalter im aufgeschobenen Zustand den Basiskörper wenigstens teilweise umgibt. Er kann aber auch z.B. in den Basiskörper einschiebbar sein.

Um eine sichere und einfache Fluidverbindung zu gewährleisten, kann der erste Basiskörper am Ende des ersten Einlassabschnitts einen sich in die erste Verbindungsrichtung erstreckenden, die erste Einlassöffnung bildenden ersten Einlassstutzen aufweisen. Dieser ist dann entlang der ersten Verbindungsrichtung in die erste Auslassöffnung des ersten Behälterhalters einschiebbar, wobei die Auslassöffnung in diesem Fall entsprechend komplementär zum Einlassstutzen ausgebildet ist. Um eine Abdichtung zwischen dem ersten Behälterhalter und dem Applikatorhalter zu gewährleisten, kann auf den Einlassstutzen ein O-Ring aufgeschoben sein, der an einer Schulter oder einem Kragen am Einlassstutzen anliegt. Selbstverständlich sind aber auch andere, dichtende Verbindungen möglich, z.B. Kegelverbindungen.

Um die Führung des Behälterhalters am Applikatorhalter zu verbessern und/oder um die Orientierung des Behälterhalters relativ zum Applikatorhalter festzulegen, kann der Basiskörper beabstandet zum ersten Einlassstutzen mindestens ein sich im Wesentlichen parallel zum ersten Einlassstutzen erstreckendes erstes Führungselement umfassen, z.B. in Form einer langgestreckten Nase oder eines Stifts. Auf das Führungselement ist dann vorzugsweise ein komplementär dazu ausgebildeter, hohler Verbindungsabschnitt des ersten Behälterhalter aufschiebbar. Auf diese Weise kann der erste Behälterhalter in einer definierten Orientierung mit dem Applikatorhalter verbunden werden und ist gegen Verdrehungen gegenüber dem Applikatorhalter gesichert. Vorzugsweise sind zwei derartige Führungselemente auf gegenüberliegenden Seiten des Einlassstutzens angeordnet und greifen entsprechend in zwei hohle Verbindungsabschnitte des Behälterhalters ein.

Um den Applikator am Befestigungsbereich des Applikatorhalters zu befestigen, kann der Befestigungsbereich ein Aufnahmeelement aufweisen, z.B. in Form eines Ringes, in das ein distaler Endbereich des Applikators einschiebbar ist und an dem eine Raststruktur, z.B. ein Rastfenster, ausgebildet ist, um mit einem korrespondierenden Rastelement des Applikators, z.B. einer Rastnase, eine lösbare Rastverbindung einzugehen. Andere Befestigungsarten sind selbstverständlich denkbar, z.B. eine normale Luerverbindung mit oder ohne Sicherungsmutter.

Bei der Befüllvorrichtung handelt es sich um eine Vorrichtung zum Befüllen eines Applikators mit zwei oder mehr parallelen Reservoirs, z.B. einer Doppel- oder Mehrfachspritze, einer Kartusche mit zwei oder mehr Reservoirs, zweier lösbar verbundener Einzelspritzen usw. Die Befüllvorrichtung umfasst (mindestens) einen zweiten Behälterhalter, mit einem zweiten Haltebereich, der dazu ausgebildet ist, einen zweiten Behälter am zweiten Behälterhalter zu halten. Der zweite Behälterhalter weist dann wiederum eine zweite Auslassöffnung und einen Fluidkanal zwischen dem zweiten Haltebereich und der zweiten Auslassöffnung auf, um ein zweites Fluid aus dem zweiten Behälter zu entnehmen. Entsprechend weist der Applikatorhalter dann ausserdem eine zweite Einlassöffnung und eine zweite Fluidverbindung zwischen der zweiten Einlassöffnung und dem Befestigungsbereich auf, um das zweite Fluid durch die zweite Einlassöffnung in ein zweites Reservoir des Applikators aufzunehmen. Der zweite Behälterhalter ist dann entlang einer zweiten Verbindungsrichtung derart mit dem Applikatorhalter verbindbar, dass die zweite Auslassöffnung und die zweite Einlassöffnung miteinander kommunizieren, um eine Verbindung vom zweiten Haltebereich zum Befestigungsbereich des Applikatorhalters herzustellen. Dabei verläuft auch die zweite Verbindungsrichtung quer zur Befestigungsrichtung für den Applikator, bevorzugt in einer zur Befestigungsrichtung senkrechten Ebene, besonders bevorzugt antiparallel zur ersten Verbindungsrichtung.

In der oben dargestellten konkreten Ausgestaltung mit einem Basiskörper mit Ober- und Unterseite und an der Unterseite angeordnetem Befestigungsbereich umfasst dann die zweite Fluidverbindung bevorzugt einen sich im Basiskörper im Wesentlichen entlang der zweiten Verbindungsrichtung von der zweiten Einlassöffnung her erstreckenden zweiten Einlassabschnitt und einen damit verbundenen, zum Befestigungsbereich führenden zweiten Auslassabschnitt, und der zweite Auslassabschnitt verläuft gewinkelt zum zweiten Einlassabschnitt und erstreckt sich in Richtung der Unterseite. Bevorzugt verlaufen der erste und der zweite Auslassabschnitt im Wesentlichen parallel zueinander und entlang der Befestigungsrichtung. Der erste und der zweite Einlassabschnitt liegen bevorzugt in einer gemeinsamen Ebene und verlaufen besonders bevorzugt kollinear zueinander, d.h. sie liegen auf derselben gedachten Geraden, und weisen in entgegengesetzte Richtungen.

In bevorzugten konkreten Ausgestaltungen sind die beiden Behälterhalter jeweils auf den Applikatorhalter aufschiebbar, um die jeweilige Einlassöffnung mit der jeweiligen Auslassöffnung zu verbinden, so dass jeder der Behälterhalter den Applikatorhalter zumindest teilweise umgibt. Am ersten und zweiten Behälterhalter können dann komplementäre Verbindungselemente ausgebildet sein, um den ersten und den zweiten Behälterhalter im aufgeschobenen Zustand miteinander zu verbinden. Hierbei kann es sich insbesondere um Rastelemente für eine Rastverbindung handeln. Statt also die Behälterhalter am Applikatorhalter zu fixieren, oder zusätzlich dazu, werden also in dieser Ausführungsform die beiden Behälterhalter aneinander fixiert und dadurch auch am Applikatorhalter gehalten.

Insbesondere falls die zweite Verbindungsrichtung antiparallel zur ersten Verbindungsrichtung verläuft, können der erste und der zweite Behälterhalter jeweils mindestens ein Rastelement aufweisen, das im aufgeschobenen Zustand in den jeweils anderen Behälterhalter eingreift, insbesondere in einen Hohlraum desselben einschiebbar ist, und eine Rastverbindung des ersten und des zweiten Behälterhalter bewirkt. Bevorzugt ist dann das Rastelement des zweiten Behälterhalters an einer dem Rastelement des ersten Behälterhalters gegenüberliegenden Seite des Applikatorhalters angeordnet.

In einer besonders kompakten und eleganten Ausführung überdecken der erste und zweite Behälterhalter im aufgeschobenen Zustand den Applikatorhalter zu einer dem Befestigungsbereich abgewandten Seite hin im Wesentlichen vollständig.

Gemäss einem weiteren Aspekt stellt die vorliegende Erfindung ein modulares Befüllsystem zur Verfügung, das es ermöglicht, einen Applikator je nach Bedarf aus verschiedenartigen Behältern zu befüllen. Ein solches Befüllsystem umfasst einen Applikatorhalter der vorstehend beschriebenen Art und zwei, drei oder mehr Behälterhalter, die zum Halten von unterschiedlichen Arten von Behältern ausgebildet sind. Die Behälterhalter können, wie oben schon angegeben, auch schon mit passenden Behältern vormontiert sein.

In anderen Worten stellt die vorliegende Erfindung ein Set zur Verfügung, umfassend eine Befüllvorrichtung der oben genannten Art und mindestens einen weiteren Behälterhalter, wobei der weitere Behälterhalter zum Halten eines andersartigen Behälters ausgebildet ist als der erste und/oder zweite Behälterhalter. Zusätzlich kann das Set auch einen passenden Applikator umfassen.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: ein erfindungsgemässes Befüllsystem in perspektivischer Ansicht;
- Fig. 2: das Befüllsystem der Fig. 1 in zentralem Längsschnitt;
- Fig. 3: eine erfindungsgemässe Befüllvorrichtung in Seitenansicht;
- Fig. 4: die Befüllvorrichtung der Fig. 3 in perspektivischer Ansicht;
- Fig. 5: eine Teilansicht einer vergrösserten Schnittdarstellung des Applikatorhalters der Fig. 2 mit daran befestigtem Applikator; und
- Fig. 6: einen Querschnitt durch die Befüllvorrichtung der Fig. 3 in der Ebene VI-VI.

### BESCHREIBUNG BEVORZUGTER AUSFÜHRUNGSFORMEN

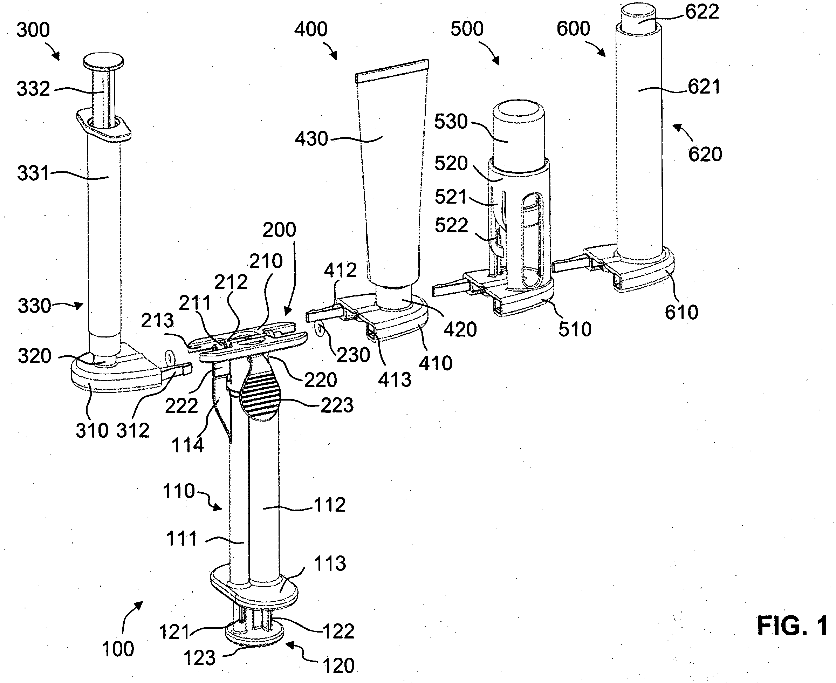

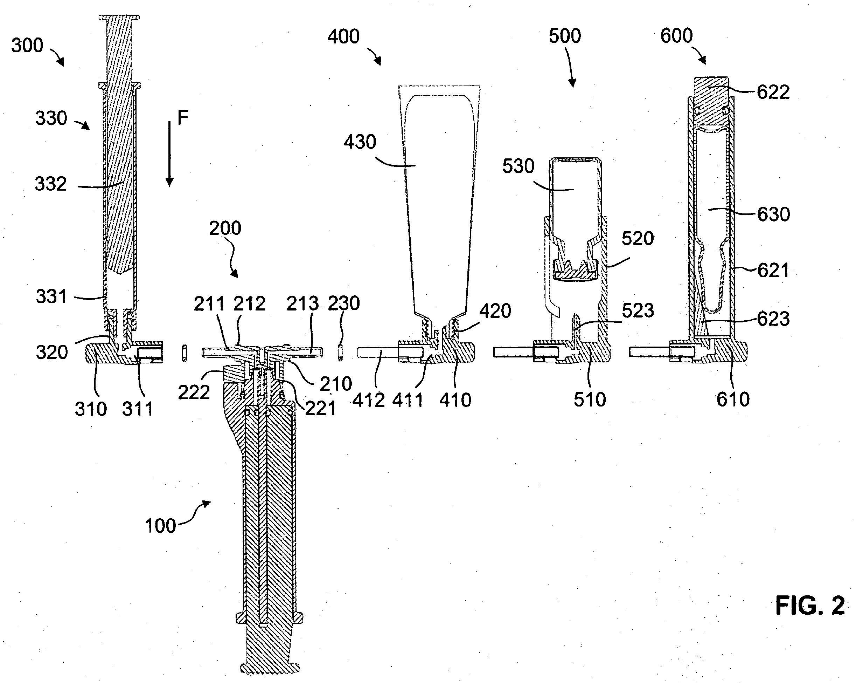

In den Figuren 1 und 2 ist ein erstes Ausführungsbeispiel eines erfindungsgemässen Befüllsystems illustriert. Das System besteht hier aus einem Applikator 100, einem Applikatorhalter 200, einer ersten Behältereinheit 300, einer zweiten Behältereinheit 400, einer dritten Behältereinheit 500 und einer vierten Behältereinheit 600.

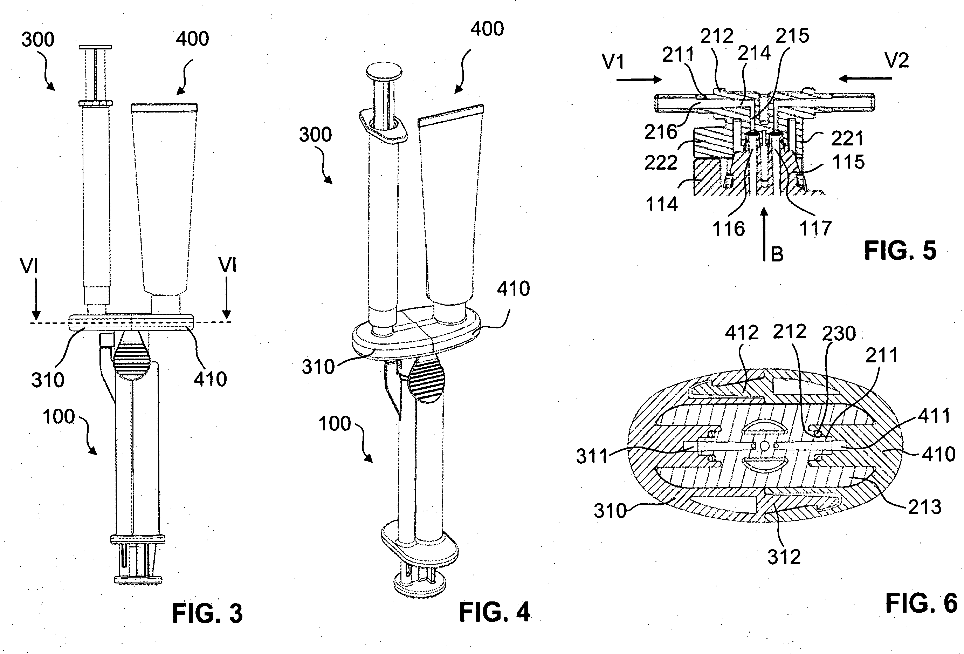

Der Applikator 100 ist doppelspritzenartig ausgebildet. Es weist einen Applikatorkörper 110 mit zwei zylindrischen, parallelen, proximal offenen Reservoirs 111, 112 gleichen oder (hier) unterschiedlichen Durchmessers und Volumens auf. Die Reservoirs münden an ihren distalen Enden in zwei Auslässe 116, 117 (Fig. 5). In die offenen proximalen Enden der Reservoirs ist jeweils ein Kolben 121, 122 eingeschoben. Die beiden Kolben sind an ihren proximalen Enden miteinander zu einer Kolbeneinheit verbunden. In diesem Bereich ist eine Betätigungsfläche 123 für den Daumen eines Benutzers ausgebildet. Ein Halteflansch 113 dient zum Halten des Applikators mittels Zeige- und Mittelfinger. Der Applikator lässt sich insoweit wie eine handelsübliche Doppelspritze handhaben.

Der Applikatorhalter 200 weist einen Basiskörper 210 von länglicher, flacher, im Wesentlichen scheibenförmiger Grundform auf, an dessen Unterseite ein Befestigungsbereich 220 für den Applikator 100 ausgebildet ist.

Der Aufbau des Basiskörpers 210 ist am besten aus den Figuren 5 und 6 erkennbar. Der Basiskörper 210 weist zwei zylindrische Einlassstutzen 211 auf, die einander gegenüberliegend kollinear auf einer gemeinsamen Längsachse des Basiskörpers angeordnet sind. Jeder der Einlassstutzen weist eine axiale Bohrung auf, deren offenes Ende eine Einlassöffnung 216 bildet und die einen Einlassabschnitt 214 einer Fluidverbindung definiert. Die beiden Einlassabschnitts 214 verlaufen somit ebenfalls kollinear zueinander. Jeder der Einlassabschnitte 214 mündet jeweils in einen Auslassabschnitt 215, der senkrecht zum jeweiligen Einlassabschnitt 214 verläuft, wobei die beiden Auslassabschnitte 215 parallel zueinander nach unten verlaufen und in den Befestigungsbereich 220 münden. Parallel zu jedem der Einlassstutzen 211 sind auf zwei Seiten des jeweiligen Einlassstutzens zwei Führungselemente in Form von Führungsstiften 213 angeordnet, deren freie Enden nach aussen hin angeschrägt sind. Die Führungsstifte ragen axial deutlich über die Einlassstutzen 211 hinaus. Auf jeden der Einlassstutzen 211 ist ein O-Ring 230 aufschiebbar, der im aufgeschobenen Zustand dichtend an einer umlaufenden Schulter 212 anliegt.

Der Befestigungsbereich 220 ist hier wie folgt ausgebildet: Jeder der Auslassabschnitte 215 mündet in einen sich konisch aufweitenden Einführbereich für die Auslässe 116, 117 des Applikators 100. Die Auslässe 116, 117 sind entsprechend komplementär zu den Einführbereichen ausgebildet und lassen sich in diese Einführbereiche einstecken. Um den Applikator 100 sicher am Applikatorhalter 200 zu halten, weist der Applikator nahe seinem distalen Ende, benachbart zu den Auslässen 116, 117, auf zwei gegenüberliegenden Seiten zwei Stege mit Einrastnasen 115 auf (Fig. 5). Der Befestigungsbereich 220 weist ein zylindrisches Aufnahmeelement 221 auf, das die Einführbereiche und die Stege mit den Einrastnasen 115 radial umgibt und an dem zwei gegenüberliegende Einschnappöffnungen ausgebildet sind. Die Einrastnasen 115 schnappen beim Einschieben des Applikators in die Einschnappöffnungen des Aufnahmeelements 221 ein. Die hier vorliegende Verbindung zwischen dem Applikator 100 und dem Applikatorhalter 200 entspricht funktional im Wesentlichen der in WO 2007/109915 beschriebenen Verbindung zwischen einer Spritze/Kartusche und einem Zubehörteil. Insbesondere umfassen der Applikator 100 und der Applikatorhalter 200 Rückhaltemittel, die gemäss diesem Dokument ausgestaltet sind.

Um den Applikator 100 nach dem Befüllen wieder vom Applikatorhalter 200 zu lösen, ist das Aufnahmeelement 221 derart elastisch verformbar ausgebildet, dass die Rastverbindung zwischen den Einrastnasen 115 und den entsprechenden Einschnappöffnungen durch Druck auf einen bezüglich der Zylinderachse des Aufnahmeelements 221 um ca. 90° von den Einschnappöffnungen versetzten Wandbereich des Aufnahmeelements 221 wieder lösbar ist. Durch einen solchen Druck wird das Aufnahmeelement 221 derartig verformt, dass die Einschnappöffnungen radial nach aussen von den Einrastnasen 115 weg gedrückt werden und dadurch ausser Eingriff mit den Einrastnasen 115 geraten. Bezüglich weiterer Details und weiterer Ausgestaltungsmöglichkeiten der Verbindung zwischen dem Applikator und dem Applikatorhalter sei auf die schon erwähnte WO 2007/109915 verwiesen, deren Inhalt bezüglich einer solchen Verbindung hierin durch Verweis aufgenommen wird.

Um den Druck auf das Aufnahmeelement 221 gezielt und einfach ausüben zu können, sind am Basiselement 210 zwei gegenüberliegende Druckflügel 223 ausgebildet. Ein seitliches Zusammendrücken der beiden Druckflügel 223 wird versetzt zu den Einschnappöffnungen auf das zylindrische Aufnahmeelement 221 des Befestigungsbereichs 220 übertragen und führt dadurch zum Lösen der Rastverbindung zwischen dem Applikator 100 und dem Applikatorhalter 200.

Ein Codierflügel 114 am Applikator 100 und ein entsprechender Codierflügel 222 am Applikatorhalter 200 zeigen die korrekte Orientierung des Applikators 100 beim Anschliessen an den Applikatorhalter 200 an. Zusätzlich sind die Anschlüsse selbst unterschiedlich ausgebildet, um sicherzustellen, dass der Applikator 100 nur in der korrekten Orientierung angeschlossen werden kann.

Die erste Behältereinheit 300 umfasst einen Behälterhalter 310 mit einem nach oben weisenden Haltebereich 320, an dem ein Behälter in Form einer Spritze 330 mit Spritzenkörper 331 und Spritzenkolben 332 gehalten ist (hierüber eine übliche Luer-Lock-Verbindung). Der Behälterhalter 310 weist eine Aussenwand auf, deren Form ganz grob einem entlang der kurzen Achsen halbierten Ellipsoids entspricht, das seitlich zum Applikatorhalter hin offen ist. Die Aussenwand begrenzt einen Innenraum, in den hinein sich ausgehend von einem Ende des halben Ellipsoids zum Applikatorhalter hin ein Anschlussbereich mit einer darin ausgebildeten Auslassöffnung 311 erstreckt. An einer Seite des Behälterhalters 310 steht ein bezüglich der Aussenwand nach innen versetzter Rastarm 312 entlang zum Applikatorhalter hin vor. Am freien Ende des Rastarms ist eine Rastnase ausgebildet. Ein um 90° gewinkelter Fluidkanal verbindet den Haltebereich 320 mit der Auslassöffnung 311.

Die zweite Behältereinheit 400 umfasst ebenfalls einen Behälterhalter 410 mit einem nach oben weisenden Haltebereich 420. Der Haltebereich ist hier als Hülse 420 mit Innengewinde ausgebildet. In den Haltebereich 420 ist ein Behälter in Form einer Tube 430 mit einem Aussengewinde an einem starrem zylindrischem Anschlussbereich eingeschraubt. Mit Ausnahme des Haltebereichs 420 ist der Behälterhalter 410 ähnlich zum Behälterhalter 310 aufgebaut und umfasst insbesondere ebenfalls eine Auslassöffnung 411, einen Fluidkanal, der den Haltebereich 420 mit der Auslassöffnung 411 verbindet, und einen Rastarm 412.

Die dritte Behältereinheit 500 umfasst wiederum einen Behälterhalter 510 mit einem nach oben weisenden Haltebereich 520, der hier in Form eines mehrfach durchbrochenen zylindrischen Kamins ausgebildet ist. In den Kamin ist ein Behälter in Form eines Vials 530 mit Septumverschluss eingeschoben. Das Vial liegt lose auf einem ersten flexiblen Haltearm 521 auf. Durch Druck auf das Vial nach unten hin lässt sich der erste Haltearm 521 über eine an im ausgebildete Schrägfläche elastisch nach aussen drücken, und das Vial lässt sich in eine Lagerposition bringen, in der es beabstandet zu einem Durchstechorgan in Form eines hohlen Doms 523 auf einem zweiten Haltearm 522, der sich weiter nach unten hin erstreckt als der erste Haltearm 521, aufliegt. In dieser Lagerposition greift der erste Haltearm 521 in eine Verjüngung des Vials ein und hindert das Vial dadurch daran, aus dem Kamin entfernt zu werden. Durch erneuten Druck nach unten hin mit erhöhter Kraft auf das Vial lässt sich auch der zweite Haltearm 522 über eine an ihm ausgebildete Schrägfläche elastisch nach aussen drücken, so dass das Vial in eine Entnahmeposition gelangt, in der der Dorn den Septumverschluss durchsticht. In der Entnahmeposition wird das Vial dann durch den zweiten flexiblen Haltearm 522 fixiert. Auf diese Weise ist es möglich, einen Behälter mit durchstechbarem Verschluss am Behälterhalter zu lagern, ohne dass der Verschluss versehentlich durchstochen werden kann oder der Behälter aus den Halter herausfallen kann. In seinem unteren Bereich ist der Behälterhalter 510 wiederum ähnlich zum Behälterhalter 310 aufgebaut.

Die vierte Behältereinheit 600 umfasst wiederum einen Behälterhalter 610 mit einem nach oben weisenden Haltebereich 620, der hier als langgestreckte zylindrische Kammer 621 mit darin dichtend verschiebbarem Kolben 622 ausgebildet ist. In der Kammer 621 ist eine Glasampulle 630 aufgenommen, die durch Druck auf den Kolben 622 auf eine Rampe 623 aufschiebbar ist, um die Ampullenspitze abzuscheren. Hiervon abgesehen ist auch der Behälterhalter 610 wiederum ähnlich zum Behälterhalter 310 aufgebaut.

Zum Befüllen der beiden Reservoirs 111, 112 wird der Applikator 100 mit vollständig eingeschobenen Kolben 121, 122 am Applikatorhalter 200 befestigt. Dazu wird der Applikator entlang einer Befestigungsrichtung B (Fig. 5) in den Befestigungsbereich 220 eingeschoben. Der Applikator kann auch schon herstellerseitig am Applikatorhalter vormontiert sein.

Auf jede der beiden gegenüberliegenden Seiten des Basiskörpers 210 wird jeweils eine Behältereinheit aufgeschoben. Im Beispiel der Figuren 3 und 4 sind dies die erste Behältereinheit 300, die entlang einer ersten Verbindungsrichtung V1 aufgeschoben wird, und die zweite Behältereinheit 400, die entlang einer zweiten Verbindungsrichtung V2 aufgeschoben wird. Die Verbindungsrichtungen V1 und V2 sind dabei antiparallel zueinander, d.h. entgegengesetzt entlang derselben Achse gerichtet, und senkrecht zur Befestigungsrichtung B.

Beim Aufschieben umgreifen die Behälterhalter 310, 410 von gegenüberliegenden Seiten her den Basiskörper 210 und überdecken seine Oberseite vollständig (Fig. 3 und 4). Auch nach unten hin ist der Basiskörper in den ausserhalb des Befestigungsbereichs 220 gelegenen Bereichen weitgehend von den beiden Behälterhaltern überdeckt. Dabei stossen die beiden Behälterhalter im aufgeschobenen Zustand aneinander an. Der Rastarm 312 des ersten Behälterhalters 310 ragt dabei in einen Einschubbereich 413 (Fig. 1) im Inneren des zweiten Behälterhalter 410 hinein, während umgekehrt auch der Rastarm 412 des zweiten Behälterhalters 410 in das Innere des ersten Behälterhalters 310 hineinragt. Jeder der Rastarme wird durch seine Rastnase in einer entsprechenden Ausnehmung auf der Innenseite der Aussenwand des jeweils anderen Behälterhalter fixiert (Fig. 6). In dieser Stellung ragt jeder der beiden Einlassstutzen 211 in die entsprechende Auslassöffnung 311, 411 des jeweiligen Behälterhalters hinein und wird gegenüber dem Behälterhalter durch den entsprechenden O-Ring 230 gedichtet. Auf diese Weise wird eine durchgehende, nach aussen hin gedichtete Fluidverbindung zwischen der Spritze 330 und dem ersten Reservoir 111 bzw. zwischen der Tube 430 und dem zweiten Reservoir 112 hergestellt.

Anschliessend wird die Kolbeneinheit 120 zurückgezogen, um die Fluide aus den beiden Behältern 330, 430 separat und gleichzeitig zu entnehmen und in die Reservoirs 111, 112 zu überführen. Dabei geht dank der kompakten Ausgestaltung der Befüllvorrichtung mit kurzen Kanälen nur eine geringe Menge des jeweiligen Fluids in der Befüllvorrichtung verloren (geringes Verlustvolumen).

Durch Druck auf die Druckflügel 223 wird nun der Applikator 100 von der Befüllvorrichtung gelöst. Anschliessend kann ein Zubehörteil, z.B. ein Mischer oder ein Sprüher, am Applikator angeschlossen werden, und die Fluide können durch das Zubehörteil hindurch aus dem Applikator ausgetragen werden.

Falls ein Fluid aus einem andersartigen Behälter in den Applikator aufgenommen werden soll, wird anstelle der ersten und/oder zweiten Behältereinheit 300, 400 einfach eine andere Behältereinheit, z.B. die dritte oder vierte Behältereinheit 500, 600 oder eine Behältereinheit mit nochmals einem andersartigen Behälter, am Applikatorhalter befestigt. Auf diese Weise ist eine grösstmögliche Freiheit in der Wahl der einzusetzenden Behälter für die Komponenten gewahrt.

Im Folgenden werden der Vollständigkeit halber noch einige Ergänzungen zum Behälterhalter 510 angebracht. Abstrakt ausgedrückt stellt der Behälterhalter 510 ein Beispiel für eine adapterartige Vorrichtung zur Entnahme eines Fluids aus einem durch einen durchstechbaren Verschluss verschlossenen Behälter zur Verfügung, die es ermöglicht, den Behälter an der Vorrichtung zu lagern, ohne den Behälter unbeabsichtigt zu öffnen. Eine solche Vorrichtung kann als eigenständiger Aspekt der vorliegenden Erfindung betrachtet werden, der unabhängig von den anderen vorstehend beschriebenen Aspekten ist.

Gemäss diesem Aspekt wird also eine Vorrichtung zum Entnehmen eines Fluids aus mindestens einem aus einem durch einen durchstechbaren Verschluss verschlossenen Behälter, insbesondere einem Vial, angegeben, die aufweist:
einen Körper, in dem eine Einlassöffnung und eine Auslassöffnung ausgebildet sind, die durch einen Fluidkanal verbunden sind;
ein mit der Einlassöffnung verbundenes hohlnadelartiges Durchstechorgan, um in einer Entnahmeposition des Behälters den Verschluss des Behälters, insbesondere einen Septumverschluss, zu durchstechen; und
eine mit dem Körper verbundene Behälteraufnahme, um den Behälter an der Vorrichtung zu halten.

Um den Behälter auch vor dem Durchstechen des Verschlusses sicher an der Vorrichtung zu halten und ein unbeabsichtigtes Durchstechen zu vermeiden, weist die Behälteraufnahme eine erste Raststruktur auf, um den Behälter in einer Lagerposition, in der der Behälter weiter vom Basiskörper entfernt ist als in der Entnahmeposition (und in der dadurch der Verschluss noch nicht durchstochen ist), durch eine lösbare Rastverbindung an der Behälteraufnahme zu fixieren. Bevorzugt ist der Behälter auch in der Entnahmeposition fixiert, und die Behälteraufnahme weist dann hierzu ausserdem eine zweite Raststruktur auf, um den Behälter in der Entnahmeposition durch eine Rastverbindung an der Behälteraufnahme zu fixieren. Die Raststrukturen können direkt oder indirekt mit dem Behälter zusammenwirken. So kann z.B. der Behälter unmittelbar in die Behälteraufnahme einschiebbar sein, wobei die Raststrukturen direkt an einer entsprechenden Rückhaltestruktur, z.B. einer Verjüngung, des Behälters angreifen, oder der Behälter kann an einem separaten Halter gehalten sein, der in die Behälteraufnahme einschiebbar oder auf diese aufschiebbar ist, wobei die Raststrukturen der Behälteraufnahme mit einer entsprechenden Rückhaltestruktur des Halters zusammenwirken. Dabei ist es auch denkbar, dass die Behälteraufnahme nur eine einzige Raststruktur aufweist, während der Behälter oder der Halter zwei Rückhaltestrukturen aufweist, wobei in der Lagerposition die erste dieser Rückhaltestrukturen mit der (einzigen) Raststruktur zusammenwirkt, während in der Entnahmeposition die zweite dieser Rückhaltestrukturen mit der Raststruktur zusammenwirkt. Jede der Raststrukturen ist bevorzugt wie folgt ausgebildet: Die Behälteraufnahme weist eine vorzugsweise zumindest teilzylindrische Mantelwand auf, die einfach oder mehrfach durchbrochen sein kann. Die erste Raststruktur und die zweite Raststruktur umfassen jeweils einen in der Mantelwand ausgebildeten Federarm, an dessen freiem Ende eine sich ins Innere der Mantelwand erstreckende Rastnase ausgebildet ist. Dies ermöglicht eine sehr einfache und kostengünstige Fertigung. Die Raststrukturen sind dabei vorzugsweise bezüglich der Umfangsrichtung der Mantelwand versetzt angeordnet, z.B. nebeneinander liegend oder um ca. 180° versetzt, d.h. einander diametral gegenüberliegend, um möglichst wenig Platz bei möglichst grosser Stabilität der Behälteraufnahme zu beanspruchen.

Während die Erfindung vorstehend anhand eines Ausführungsbeispiels beschrieben wurde, ist die Erfindung keinesfalls auf das vorstehende Ausführungsbeispiel beschränkt, und eine Vielzahl von Abwandlungen ist möglich. So kann statt eines Applikators der hier angegebenen Art selbstverständlich auch eine andere Art von Applikator eingesetzt werden, z.B. Applikatoren, wie sie in WO 2009/144085 oder WO 2007/109915 illustriert sind. Auch herkömmliche Doppelspritzen oder zu einer Einheit verbundene Einzelspritzen können eingesetzt werden. Dementsprechend ist es auch möglich, den Applikatoranschluss entsprechend anders auszugestalten. Insbesondere kann natürlich der Abstand der Auslässe des Applikators je nach Bedarf anders, insbesondere grösser, gewählt werden als in den hier illustrierten Ausführungsbeispielen. Während die hier dargestellte Art der Befestigung des Applikators am Applikatorhalter vorteilhaft ist, kann auch eine andere Art der Verbindung zwischen Befüllvorrichtung und Applikator gewählt werden, z.B. eine herkömmliche Luerverbindung. In anderen Ausgestaltungen kann der Applikator nur ein einziges Reservoir aufweisen, und es kann dementsprechend dann nur ein einziger Behälterhalter vorhanden sein. Eine Vielzahl weiterer Abwandlungen ist möglich.

**BEZUGSZEICHENLISTE**

| | | | |
|---|---|---|---|
| 100 | Applikator | 311 | Auslassöffnung |
| 110 | Applikatorkörper | 312 | Rastarm |
| 111 | erstes Reservoir | 320 | Haltebereich |
| 112 | zweites Reservoir | 330 | Spritze |
| 113 | Halteflansch | 331 | Spritzenkörper |
| 114 | Codierflügel | 332 | Spritzenkolben |
| 115 | Einrastnase | 400 | zweite Behältereinheit |
| 116 | erster Auslass | 410 | Behälterhalter |
| 117 | zweiter Auslass | 411 | Auslassöffnung |
| 120 | Kolbeneinheit | 412 | Rastarm |
| 121 | erster Kolben | 413 | Einschubbereich |
| 122 | zweiter Kolben | 420 | Haltebereich |
| 123 | Betätigungsfläche | 430 | Tube |
| 200 | Applikatorhalter | 500 | dritte Behältereinheit |
| 210 | Basiskörper | 510 | Behälterhalter |
| 211 | Anschlussstutzen | 520 | Haltebereich |
| 212 | Schulter | 521 | erster Haltearm |
| 213 | Führungsstift | 522 | zweiter Haltearm |
| 214 | Einlassabschnitt | 523 | Dorn |
| 215 | Auslassabschnitt | 530 | Vial |
| 216 | Einlassöffnung | 600 | vierte Behältereinheit |
| 220 | Befestigungsbereich | 610 | Behälterhalter |
| 221 | Aufnahmeelement | 620 | Haltebereich |
| 222 | Codierflügel | 621 | Hohlzylinder |
| 223 | Druckflügel | 622 | Kolben |
| 300 | erste Behältereinheit | 623 | Rampe |
| 310 | Behälterhalter | 630 | Ampulle |

## Patentansprüche

1. Befüllvorrichtung zum Befüllen mindestens eines ersten Reservoirs (111) eines Applikators (100) mit einem Fluid aus mindestens einem ersten Behälter (330), wobei die Befüllvorrichtung aufweist:
einen ersten Behälterhalter (310) mit einem ersten Haltebereich (320), der dazu ausgebildet ist, einen ersten Behälter (330) am ersten Behälterhalter (310) zu halten, wobei der erste Behälterhalter (310) eine erste Auslassöffnung (311) und einen Fluidkanal zwischen dem ersten Haltebereich und der ersten Auslassöffnung aufweist, um ein erstes Fluid durch die erste Auslassöffnung (311) hindurch aus dem ersten Behälter (330) zu entnehmen; und
einen Applikatorhalter (200) mit einem Befestigungsbereich (220), der dazu ausgebildet ist, einen Applikator (100) entlang einer Befestigungsrichtung (B) lösbar am Applikatorhalter (200) zu befestigen, und wobei der Applikatorhalter (200) eine erste Einlassöffnung (216) und eine erste Fluidverbindung zwischen der ersten Einlassöffnung (216) und dem Befestigungsbereich (220) aufweist, um das erste Fluid durch die erste Einlassöffnung (216) in ein erstes Reservoir (111) des Applikators (100) aufzunehmen;
wobei der erste Behälterhalter (310) entlang einer ersten Verbindungsrichtung (V1) derart mit dem Applikatorhalter (200) verbindbar ist, dass die erste Auslassöffnung (311) und die erste Einlassöffnung (216) miteinander kommunizieren, und
wobei die erste Verbindungsrichtung (V1) quer zur Befestigungsrichtung (B) für den Applikator (100) verläuft,
**dadurch gekennzeichnet, dass** die Befüllvorrichtung einen zweiten Behälterhalter (410) umfasst, mit einem zweiten Haltebereich (420), der dazu ausgebildet ist, einen zweiten Behälter (430) am zweiten Behälterhalter zu halten, wobei der zweite Behälterhalter (410) eine zweite Auslassöffnung (411) und einen Fluidkanal zwischen dem zweiten Haltebereich (420) und der zweiten Auslassöffnung (411) aufweist, um ein zweites Fluid aus dem zweiten Behälter (430) zu entnehmen,
wobei der Applikatorhalter (200) ausserdem eine zweite Einlassöffnung und eine zweite Fluidverbindung zwischen der zweiten Einlassöffnung und dem Befestigungsbereich aufweist, um das zweite Fluid durch die zweite Einlassöffnung in ein zweites Reservoir (112) des Applikators (100) aufzunehmen,
wobei der zweite Behälterhalter (410) entlang einer zweiten Verbindungsrichtung (V2) derart mit dem der Applikatorhalter (200) verbindbar ist, dass die zweite Auslassöffnung (411) und die zweite Einlassöffnung miteinander kommunizieren, um eine Verbindung vom zweiten Haltebereich (420) zum Befestigungsbereich (220) des Applikatorhalters (200) herzustellen, wobei die zweite Verbindungsrichtung (V2) quer zur Befestigungsrichtung (B) für den Applikator verläuft.

2. Befüllvorrichtung nach Anspruch 1, wobei der erste Behälter (330) entlang einer Behälterführungsrichtung (F) am ersten Behälterhalter (310) anbringbar ist, die quer zur ersten Verbindungsrichtung (V1) verläuft und die im Wesentlichen antiparallel zur Befestigungsrichtung (B) für den Applikator (100) verläuft, wenn der Applikatorhalter (200) und der erste Behälterhalter (310) miteinander verbunden sind.

3. Befüllvorrichtung nach Anspruch 1 oder 2, wobei der erste Behälterhalter (310) dazu ausgebildet ist, eine Spritze, eine Tube, ein Vial mit durchstechbarem Verschluss oder eine Ampulle zu halten.

4. Befüllvorrichtung nach einem der vorhergehenden Ansprüche,
wobei der Applikatorhalter (200) einen Basiskörper (210) mit einer Oberseite und einer Unterseite umfasst,
wobei der Befestigungsbereich (220) an der Unterseite des Basiskörpers (210) angeordnet ist,
wobei die erste Fluidverbindung einen sich im Basiskörper (210) im Wesentlichen entlang der ersten Verbindungsrichtung (V1) von der ersten Einlassöffnung (216) her erstreckenden ersten Einlassabschnitt (214) und einen damit verbundenen, zum Befestigungsbereich (220) führenden ersten Auslassabschnitt (215) aufweist, und
wobei der erste Auslassabschnitt (215) gewinkelt zum ersten Einlassabschnitt (214) verläuft und sich in Richtung der Unterseite erstreckt.

5. Befüllvorrichtung nach Anspruch 4, wobei der erste Behälterhalter (310) entlang der ersten Verbindungsrichtung (V1) auf den Basiskörper (210) aufschiebbar ist, um die erste Einlassöffnung (216) mit der ersten Auslassöffnung (311) zu verbinden.

6. Befüllvorrichtung nach Anspruch 4 oder 5, wobei der Basiskörper (210) am Ende des ersten Einlassabschnitts (214) einen sich in die erste Verbindungsrichtung (V1) erstreckenden, die erste Einlassöffnung (216) bildenden ersten Einlassstutzen (211) aufweist, der entlang der ersten Verbindungsrichtung (V1) in die komplementär dazu ausgebildete Auslassöffnung (311) des ersten Behälterhalters (310) einschiebbar ist.

7. Befüllvorrichtung nach Anspruch 6, wobei am ersten Einlassstutzen (211) eine umlaufende Schulter (212) ausgebildet ist, und wobei auf den ersten Einlassstutzen (211) ein O-Ring (230) aufgeschoben ist, so dass der O-Ring (230) an der Schulter (212) anliegt.

8. Befüllvorrichtung nach Anspruch 6 oder 7, wobei der Basiskörper (210) beabstandet zum ersten Einlassstutzen (211) mindestens ein sich im Wesentlichen parallel zum ersten Einlassstutzen (211) erstreckendes erstes Führungselement (213) umfasst, auf das ein komplementär dazu ausgebildeter hohler Verbindungsabschnitt des ersten Behälterhalters (310) aufschiebbar ist, um den ersten Behälterhalter (310) in einer definierten Orientierung mit dem Applikatorhalter (200) zu verbinden.

9. Befüllvorrichtung nach einem der vorhergehenden Ansprüche, wobei der Befestigungsbereich (220) des Applikatorhalters (200) ein Aufnahmeelement (221) aufweist, in das ein distaler Endbereich des Applikators (100) einschiebbar ist und an dem eine Raststruktur ausgebildet ist, um mit einem korrespondierenden Rastelement des Applikators (100) eine lösbare Rastverbindung einzugehen.

10. Befüllvorrichtung nach einem der vorhergehenden Ansprüche,
wobei der Applikatorhalter einen Basiskörper (210) mit einer Oberseite und einer Unterseite umfasst,
wobei der Befestigungsbereich (220) an der Unterseite des Basiskörpers (210) angeordnet ist,
wobei die erste Fluidverbindung einen sich im Basiskörper (210) im Wesentlichen entlang der ersten Verbindungsrichtung (V1) von der ersten Einlassöffnung her erstreckenden ersten Einlassabschnitt (214) und einen damit verbundenen, zum Befestigungsbereich (220) führenden ersten Auslassabschnitt (215) aufweist,
wobei der erste Auslassabschnitt (215) gewinkelt zum ersten Einlassabschnitt (214) verläuft und sich in Richtung der Unterseite erstreckt,
wobei die zweite Fluidverbindung einen sich im Basiskörper (210) im Wesentlichen entlang der zweiten Verbindungsrichtung (V2) von der zweiten Einlassöffnung her erstreckenden zweiten Einlassabschnitt und einen damit verbundenen, zum Befestigungsbereich führenden zweiten Auslassabschnitt aufweist, und
wobei zweite Auslassabschnitt gewinkelt zum zweiten Einlassabschnitt verläuft und sich in Richtung der Unterseite erstreckt.

11. Befüllvorrichtung nach einem der vorhergehenden Ansprüche, wobei die zweite Verbindungsrichtung (V2) antiparallel zur ersten Verbindungsrichtung (V1) verläuft.

12. Befüllvorrichtung nach einem der vorhergehenden Ansprüche,
wobei der erste Behälterhalter (310) entlang der ersten Verbindungsrichtung (V1) auf den Applikatorhalter aufschiebbar ist, um die erste Einlassöffnung (216) mit der ersten Auslassöffnung (311) zu verbinden,
wobei der zweite Behälterhalter (410) entlang der zweiten Verbindungsrichtung (V2) auf den Applikatorhalter (200) aufschiebbar ist, um die zweite Einlassöffnung mit der zweiten Auslassöffnung (411) zu verbinden,
und wobei am ersten und zweiten Behälterhalter komplementäre Verbindungselemente ausgebildet sind, um den ersten und den zweiten Behälterhalter im aufgeschobenen Zustand miteinander zu verbinden.

13. Befüllvorrichtung nach Anspruch 12, wobei die zweite Verbindungsrichtung (V2) antiparallel zur ersten Verbindungsrichtung (V1) verläuft und wobei der erste und der zweite Behälterhalter jeweils mindestens ein Rastelement (312; 412) aufweisen, das im aufgeschobenen Zustand in den jeweils anderen Behälterhalter eingreift und eine Rastverbindung des ersten und des zweiten Behälterhalters bewirkt.

14. Befüllvorrichtung nach Anspruch 12 oder 13, wobei der erste und zweite Behälterhalter im aufgeschobenen Zustand den Applikatorhalter (200) zu einer dem Befestigungsbereich (220) abgewandten Seite hin im Wesentlichen vollständig überdecken.

15. Set, umfassend eine Befüllvorrichtung nach einem der vorhergehenden Ansprüche und mindestens einen weiteren Behälterhalter (510; 610), wobei der weitere Behälterhalter (510; 610) zum Halten eines andersartigen Behälters ausgebildet ist als der erste Behälterhalter.

## Claims

1. A filling device for filling at least a first reservoir (111) of an applicator (100) with fluid from at least a first container (330), the filling device comprising:
a first container holder (310) with a first holding area (320) which is designed to hold a first container (330) on the first container holder (310), the first container holder (310) having a first outlet opening (311) and a fluid channel between the first holding area and the first outlet opening in order to remove a first fluid from the first container (330) through the first outlet opening (311); and
an applicator holder (200) with a fastening area (220) which is designed for removably fastening an applicator (100) to the applicator holder (200) along a fastening direction (B), the applicator holder (200) having a first inlet opening (216) and a first fluid connection between the first inlet opening (216) and the fastening area (220) in order to take up the first fluid into a first reservoir (111) of the applicator (100) through the first inlet opening (216);
wherein the first container holder (310) is configured to be connected to the applicator holder (200) along a first connection direction (V1) in such a way that the first outlet opening (311) and the first inlet opening (216) are in communication with each other, and
wherein the first connection direction (V1) is transverse to the fastening direction (B) for the applicator (100),
**characterised in that** the filling device comprises a second container holder (410) with a second holding area (420) which is designed to hold a second container (430) on the second container holder, wherein the second container holder (410) has a second outlet opening (411) and a fluid channel between the second holding area and the second outlet opening (411) in order to remove a second fluid from the second container (430),
wherein the applicator holder (200) further has a second inlet opening and a second fluid connection between the second inlet opening and the fastening area in order to take up the second fluid into a second reservoir (112) of the applicator (100) through the second inlet opening (216),
wherein the second container holder (410) is configured to be connected to the applicator holder (200) along a second connection direction (V2) in such a way that the second outlet opening (411) and the second inlet opening are in communication with each other in order to create a connection from the second holding area (420) to the fastening area (220) of the applicator holder (200),
wherein the second connection direction (V2) is transverse to the fastening direction (B) for the applicator.

2. The filling device in accordance with claim 1, wherein the first container (330) can be attached to the first container holder (310) along a container guiding direction (F) which runs transversely to the first connection direction (V1) and essentially runs antiparallel to the fastening direction (B) for the applicator (100) when the applicator holder (200) and the first container holder (310) are connected to each other.

3. The filling device in accordance with claim 1 or 2, wherein the first container holder (310) is designed to hold a syringe, a tube, a vial with a closure that can be punctured, or an ampoule.

4. The filling device in accordance with any one of the preceding claims,
wherein the applicator holder (200) comprises a basic body (210) with an upper side and an underside,
wherein the fastening area (220) is arranged on the underside of the basic body (210),
wherein the first fluid connection has a first inlet section (214) essentially extending along the first connection direction (V1) from the first inlet opening (216) and connected thereto a first outlet section (215) leading to the fastening area (220), and
wherein the first outlet section (215) runs at an angle to the first inlet section (214) and extends toward the underside.

5. The filling device in accordance with claim 4, wherein the first container holder (310) is configured to be pushed onto the basic body (210) along the first connection direction (V1) in order to connect the first inlet opening (216) to the first outlet opening (311).

6. The filling device in accordance with claim 4 or 5, wherein at the end of the first inlet section (214) the basic body (210) has a first inlet connection piece (211), extending in the first connection direction (V1) and forming the first inlet opening, the first inlet connection piece being configured to be pushed into the complementary outlet opening (311) of the first container holder (310) in the first connection direction (V1).

7. The filling device in accordance with claim 6, wherein on the first inlet connection piece (211) a circumferential shoulder (212) is formed and wherein an O-ring (230) is pushed onto the first inlet connection piece (211) so that the O-ring (230) adjoins the shoulder (212).

8. The filling device in accordance with claim 6 or 7, wherein at a distance from the first inlet connection piece (211) the basic body (210) has at least one first guide element (213), essentially extending parallel to the first inlet connection piece (211), onto which a complementary hollow connection section of the first container holder (310) is configured to be pushed in order to connect the first container holder (310) to the applicator holder (200) in a defined orientation.

9. The filling device in accordance with any one of the preceding claims, wherein the fastening area (220) of the applicator holder (200) has a receiving element (221) into which a distal end area of the applicator (100) can be pushed and on which a catch structure is formed in order to bring about a releasable snap-type connection with a corresponding engaging element of the applicator (100).

10. The filling device in accordance with any one of the preceding claims,
wherein the applicator holder comprises a basic body (210) with an upper side and an underside;
wherein the fastening area (220) is arranged on the underside of the basic body (210);
wherein the first fluid connection has a first inlet section (214) in the basic body (210) essentially extending along the first connection direction (V1) from the first inlet opening (216) and connected thereto a first outlet section (215) leading to the fastening area (220);
wherein the first outlet section (215) runs at an angle to the first inlet section (214) and extends toward the underside;
wherein the second fluid connection has a second inlet section in the basic body (210) essentially extending along the second connection direction (V2) from the second inlet opening and connected thereto a second outlet section leading to the fastening area; and
wherein the second outlet section runs at an angle to the second inlet section and extends toward the underside.

11. The filling device in accordance with any one of the preceding claims, wherein the second connection direction (V2) runs antiparallel to the first connection direction (V1).

12. The filling device in accordance with any one of the preceding claims,
wherein the first container holder (310) is configured to be pushed onto the applicator holder along the first connection direction (V1) in order to connect the first inlet opening (216) to the first outlet opening (311),
wherein the second container holder (410) is configured to be pushed onto the applicator holder (200) along the second connection direction (V2) in order to connect the second inlet opening to the second outlet opening (211),
and wherein complementary connection elements are formed on the first and second container holders in order to connect the first and the second container holders to each other in the mounted state.

13. The filling device in accordance with claim 12, wherein the second connection direction (V2) runs antiparallel to the first connection direction (V1) and wherein the first and the second container holders each have at least one engaging element (312; 412) which in the mounted state engages in the other container holder and brings about a snap-type connection between the first and the second container holder.

14. The filling device in accordance with claim 12 or 13, wherein the first and the second container holders in the mounted state essentially completely cover the application holder (200) towards a side facing away from the fastening section (220).

15. A set comprising a filling device in accordance with any one of the preceding claims and at least one further container holder (510; 610), wherein the further container holder (510; 610) is designed for holding a container designed differently from the first container holder.

## Revendications

1. Un dispositif de remplissage pour remplir au moins un premier réservoir (111) d'un applicateur (100) avec un fluide d'un au moins premier récipient (330), le dispositif de remplissage comprenant:
un premier support de récipient (310) avec une première zone de retenue (320) qui est conçu pour retenir un premier récipient (330) sur le premier support de récipient (310), le premier support de récipient (310) ayant une première ouverture de sortie (311) et un canal de fluide entre la première zone de retenue et la première ouverture de sortie afin retirer un premier fluide du premier récipient (330) à travers la première ouverture de sortie (311); et
un support d'applicateur (200) avec une zone de fixation (220) qui est conçu pour fixer de façon amovible un applicateur (100) sur le support d'applicateur (200) le long d'une direction de fixation (B),et le support d'applicateur (200) présentant une première ouverture d'entrée (216) et une première connexion de fluide entre la première ouverture d'entrée (216) et la zone de fixation (220), afin de recevoir le premier fluide dans un premier réservoir (111) de l'applicateur (100) à travers la première ouverture d'entrée (216);
où le premier support de récipient (310) est configuré pour être connecté au support d'applicateur (200) le long d'une première direction de connexion (V1) d'une telle manière que la première ouverture de sortie (311) et la première ouverture d'entrée (216) sont en communication l'une avec l'autre, et
où la première direction de connexion (V1) est transversale à la direction de fixation (B) pour l'applicateur (100),
**caractérisé en ce que** le dispositif de remplissage comprend un deuxième support de récipient (410) avec une deuxième zone de retenue (420) qui est conçu pour retenir un deuxième récipient (430) sur le deuxième support de récipient,
où le deuxième support de récipient (410) présente une deuxième ouverture de sortie (411) et un canal de fluide entre la deuxième zone de retenue et la deuxième ouverture de sortie (411) afin de retirer un deuxième fluide du deuxième récipient (430),
où le support d'applicateur (200) présente en outre une deuxième ouverture d'entrée et une deuxième connexion de fluide entre la deuxième ouverture d'entrée et la zone de fixation, afin de recevoir le deuxième fluide dans un deuxième réservoir (112) de l'applicateur (100) à travers la deuxième ouverture d'entrée (216),
où le deuxième support de récipient (410) est configuré pour être connecté au support d'applicateur (200) le long d'une deuxième direction de connexion (V2) de telle manière que la deuxième ouverture de sortie (411) et la deuxième ouverture d'entrée sont en communication l'une avec l'autre afin de créer une connexion depuis la deuxième zone de retenue (420) vers la zone de fixation (220) du support d'applicateur (200),
où la deuxième direction de connexion (V2) est transversale à la direction de fixation (B) pour l'applicateur.

2. Le dispositif de remplissage selon la revendication 1, où le premier récipient (330) peut être fixé sur le premier support de récipient (310) le long d'une direction de guidage du conteneur (F) qui s'étend transversalement par rapport à la première direction de connexion (V1) et essentiellement s'étend antiparallèle à la direction de fixation (B) pour l'applicateur (100) lorsque le support d'applicateur (200) et le premier support de récipient (310) sont reliés l'un à l'autre.

3. Le dispositif de remplissage selon la revendication 1 ou 2, où le premier support de récipient (310) est conçu pour recevoir une seringue, un tube, un flacon avec un bouchon transperçable ou une ampoule.

4. Le dispositif de remplissage selon l'une quelconque des revendications précédentes,
où le support d'applicateur (200) comprend un corps de base (210) avec un côté supérieur et un côté inférieur,
où la zone de fixation (220) est disposé sur le côté inférieur du corps de base (210),
où la première connexion de fluide comprend une première section d'entrée (214) se prolongeant dans le corps de base (210) essentiellement le long de la première direction de connexion (V1) depuis la première ouverture d'entrée (216) et connectée à celle-ci une première section de sortie (215) menant à la zone de fixation (220), et
où la première section de sortie (215) s'étend à un angle par rapport à la première section d'entrée (214) et se prolonge en direction du côté inférieur.

5. Le dispositif de remplissage selon la revendication 4, où le premier support de récipient (310) est configuré pour être poussé sur le corps de base (210) le long de la première direction de connexion (V1) afin de connecter la première ouverture d'entrée (216) à la première ouverture de sortie (311).

6. Le dispositif de remplissage selon la revendication 4 ou 5, où à la fin de la première section d'entrée (214) le corps de base (210) présente une première pièce de raccordement d'entrée (211), s'étendant dans la première direction de connexion (V1) et formant la première ouverture d'entrée, la première pièce de raccordement d'entrée étant configuré pour être poussé dans l'ouverture de sortie y complémentaire (311) du premier support de récipient (310) dans la première direction de connexion (V1).

7. Le dispositif de remplissage selon la revendication 6, où sur la première pièce de raccordement d'entrée (211), un épaulement périphérique (212) est formé et où un joint torique (230) est poussé sur la première pièce de raccordement d'entrée (211) de sorte que le joint torique (230) jouxte l'épaulement (212).

8. Le dispositif de remplissage selon la revendication 6 ou 7, où à une certaine distance de la première pièce de raccordement d'entrée (211) le corps de base (210) comporte au moins un premier élément de guidage (213), s'étendant essentiellement parallèlement à la première pièce de raccordement d'entrée (211) sur lequel une section de connexion creuse y complémentaire du premier support de récipient (310) est configurée pour être poussée afin de connecter le premier support de récipient (310) au support d'applicateur (200) dans une orientation définie.

9. Le dispositif de remplissage selon une des revendications précédentes, où la zone de fixation (220) du support d'applicateur (200) présente un élément de réception (221) dans lequel une zone d'extrémité distale de l'applicateur (100) peut être poussé et sur lequel une structure de capture est formée de manière à entraîner une connexion du type à encliquetage libérable avec un élément d'engagement correspondant de l'applicateur (100).

10. Le dispositif de remplissage selon une des revendications précédentes,
où le support d'applicateur comprend un corps de base (210) avec un côté supérieur et un côté inférieur;
où la zone de fixation (220) est disposée sur le côté inférieur du corps de base (210);
où la première connexion de fluide comprend une première section d'entrée (214) s'étendant dans le corps de base (210) essentiellement le long de la première direction de connexion (V1) depuis la première ouverture d'entrée et connectée à celle-ci une première section de sortie (215) menant à la zone de fixation (220);
où la première section de sortie (215) tourne à un angle par rapport à la première section d'entrée (214) et se prolonge vers le côté inférieur;
où la deuxième connexion de fluide présente une deuxième section d'entrée dans le corps de base (210) s'étendant essentiellement le long de la deuxième direction de connexion (V2) depuis la deuxième ouverture d'entrée et connectée à celle-ci une deuxième section de sortie menant à la zone de fixation; et
où la deuxième section de sortie s'étend à un angle par rapport à la deuxième section d'entrée et se prolonge vers le côté inférieur.

11. Le dispositif de remplissage selon une des revendications précédentes, où la deuxième direction de connexion (V2) s'étend antiparallèlement à la première direction de connexion (V1).

12. Le dispositif de remplissage selon une des revendications précédentes,
où le premier support de récipient (310) est configuré pour être poussé sur le support d'applicateur le long de la première direction de connexion (V1) afin de connecter la première ouverture d'entrée (216) à la première ouverture de sortie (311),
où le deuxième support de récipient (410) est configuré pour être poussé sur le support d'applicateur (200) le long de la deuxième direction de connexion (V2) afin de connecter la deuxième ouverture d'entrée à la deuxième ouverture de sortie (411), et
où les éléments de connexion complémentaires sont formés sur les premiers et deuxièmes supports de récipient afin de connecter le premier et le deuxième support de récipient l'un à l'autre dans l'état monté.

13. Le dispositif de remplissage selon la revendication 12, où la deuxième direction de connexion (V2) s'étend antiparallèlement à la première direction de connexion (V1) et où le premier et le deuxième support de récipient présentent chacun au moins un élément d'engagement (312; 412) qui, dans l'état monté, vient en prise dans l'autre support de récipient et provoque une liaison de type encliquetage entre le premier et le deuxième support de récipient.

14. Le dispositif de remplissage selon la revendication 12 ou 13, où les premiers et les deuxièmes supports de récipient dans l'état monté couvrent essentiellement complètement le support d'applicateur (200) en direction d'un côté opposé à la section de fixation (220).

15. Un ensemble comprenant un dispositif de remplissage selon l'une quelconque des revendications précédentes et au moins un autre support de récipient (510; 610), où l'autre support de récipient (510; 610) est conçue pour maintenir un récipient conçu différemment du premier support de récipient.
